# EUROPEAN PATENT SPECIFICATION

(11) **EP 2 568 964 B1**
(45) Date of publication and mention of the grant of the patent: **01.08.2018**
(21) Application number: 11706248.9
(22) Date of filing: 03.03.2011
(51) Int. Cl.: A61K 9/14, A61K 31/427, A61K 9/16, A61K 9/48

(54) **PHARMACEUTICAL DOSAGE FORM COMPRISING ONE OR MORE ANTIRETROVIRAL ACTIVE INGREDIENTS**
PHARMAZEUTISCHE DARREICHUNGSFORM MIT EINEM ODER MEHREREN ANTIRETROVIRALEN WIRKSTOFFEN
FORME POSOLOGIQUE PHARMACEUTIQUE COMPRENANT UN OU PLUSIEURS PRINCIPES ACTIFS ANTIRÉTROVIRAUX

(30) Priority: 10.05.2010 IN 1306CH2010
(43) Date of publication of application: 20.03.2013
(73) Proprietor: Evonik Röhm GmbH, 64293 Darmstadt (DE)
(72) Inventor: NALAWADE, Pravin, Thane 421503 (IN); SHETTY, Smitha, (East) Mumbai 400101 (IN); RAVISHANKAR, Hema, Nassim Ville 258378 (SG); GADHINGLAJKAR, Shripad, Thane (West) 400607 (IN); GRYCZKE, Andreas, 64560 Riedstadt (DE); PETEREIT, Hans-Ulrich, 64291 Darmstadt (DE); NOLLENBERGER, Kathrin, 60598 Frankfurt (DE)
(86) International application number: PCT/EP2011/053137
(87) International publication number: WO 2011/141192

(56) References cited:
- EP-A1- 2 279 728
- WO-A1-2004/019918
- WO-A1-2004/066976
- WO-A2-2008/017867
- WO-A2-2008/067164

## Description

### Field of the invention

The invention belongs to the field of pharmaceutical dosage forms comprising one or more antiretroviral active ingredient belonging to the BCS class III or to the BCS class IV in the form of a solid dispersion or solid solution in a matrix.

### Technical Background

US 6,391,338 B1 describes a system for rendering substantially non-dissoluble bio-affecting agents bio-available. The compositions are comprising a solid substantially uniform dispersion of a bio-affecting agent and a water-soluble polymer, wherein said bio-affecting agent is fixed in an increased-energy state by said polymer, whereby said agent is released in solution in the form of nano-particles. For instance bio-affecting agents like ibuprofen may be mixed in an extrusion process with EUDRAGIT® E as water-soluble polymer.

EP 1302 201A1 describes a pharmaceutical composition for oral use with improved absorption, which comprises drug, aminoalkyl mehtacrylate copolymer E and acidic substance, where said 3 components are brought together and at least the above-mentioned polymer and above-mentioned acidic substance are uniformly mixed. Among the long list of possibly suitable acidic substances also C12-C18 mono carboxylic acids like for instance stearic acid are mentioned. In the examples hydrochloric acid (HCl), citric acid, malic acid and tartaric acid are combined with EUDRAGIT® E or EUDRAGIT® EPO.

US 7,175,857 (WO2004/019918) describes a process for the production of granules or powders, suitable as coating agents and binders for oral or dermal pharmaceutical forms, for cosmetics or food supplements, consisting essentially of (a) a copolymer, consisting of free radical-polymerized C₁- to C₄-esters of acrylic or methacrylic acid and further (meth)acrylate monomers which contain functional tertiary amino groups, (b) 3 to 25% by weight, based on (a), of an emulsifier having an HLB of at least 14, (c) 5 to 50% by weight, based on (a), of a C₁₂- to C₁₈-monocarboxylic acid or of a C₁₂-to C₁₈-hydroxyl compound, where the components (a), (b) and (c) are simultaneously or successively blended or mixed with one another, optionally with addition of a pharmaceutical active compound and/or further customary additives, fused in a heatable mixer, mixed, the melt is cooled and comminuted to give granules or powders. The examples describe the melt extrusion of mixtures of EUDRAGIT® E with stearic acid and sodium lauryl sulfate. One of the objects of the invention is to avoid dust formation during further processing.

US20060051412A1 (WO2004066976A1) describes a method for producing an oral pharmaceutical form with immediate disintegration and active ingredient release even in the mouth, by vigorously mixing (a) an anionic active pharmaceutical ingredient with (b) a copolymer consisting of free-radical polymerized C₁ to C₄ esters of acrylic or methacrylic acid and further (meth)acrylate monomers which have functional tertiary amino groups, and (c) 5 to 50% by weight, based on (b), of a C₁₂ to C₂₂ carboxylic acid in the melt, solidifying the mixture and grinding to an active ingredient-containing powder with an average particle size of 200 µm or less, incorporating the powder into a water-soluble matrix of pharmaceutically customary excipients, with the proviso that not more than 3% by weight, based on the copolymer, of emulsifiers having an HLB of at least 14 may be present. The examples describe the melt extrusion of mixtures of ibuprofen, EUDRAGIT® E and stearic acid. The invention is based on the interaction of the anionic active ingredients with the cationic copolymers.

WO2008/017867A2 describes antiretroviral solid oral compositions. The composition comprises one or more anitretroviral drugs such as lopinavir and ritonavir and a water insoluble polymer. The water insoluble polymer may be EUDRAGIT® E. In an example a mixture of Kollidon® VA64 (POLYVINYL PYRROLIDONE:vinyl acetate) and EUDRAGIT® E with lopinavir and ritonavir is applied. The ratio of drug to polymer ranges in general from 1 : 1 to about 1 : 6. Further excipients may be contained; however mono carboxylic acids or alcohols with 12 to 22 carbon atoms are not mentioned. The compositions are preferably produced by melt extrusion at temperatures ranging from 70 to 200 °C. It is mentioned that a person skilled in the art will appreciate that melt extrusion with certain water insoluble polymers leads to an increase in solubility of poorly soluble drugs.

WO2008/067164A2 describes solid pharmaceutical dosage forms comprising a solid dispersion or solid solution of ritonavir in a matrix. The matrix comprises at least one pharmaceutical acceptable water-soluble polymer and at least one pharmaceutically acceptable surfactant. The pharmaceutically acceptable surfactant has an HLB value of from 12 to 18. The most preferred water-soluble polymer seems to be N-vinyl pyrrolidone, however a lot of other polymers among them butyl methacrylate/2-dimethylaminoethyl methacrylate copolymers are also generally mentioned to be suitable. Further excipients such as mono carboxylic acids or alcohols with 12 to 22 carbon atoms are not mentioned. The preferred technique to produce the solid dispersion or solid solution of ritonavir in the matrix is melt extrusion. Usual temperature are in the range of 70 to 250 °C, preferably 80 to 180 °C or most preferred 100 to 140 °C.

Gelderblom et al. (Cremophor® EL: The drawbacks and advantages of vehicle selection for drug fomulation (2001) Gelderblom, H., Verweij J. and Sparreboom A., European Journal of Cancer, 37, 1590 - 1598) discuss the biological effects of Cremophor® EL as an often used formulation vehicle in connection the development of anti cancer agents.

Lorenz et al. (Histamine release and hyposensitive reactions in dogs by solubilizing agents and fatty acids: Analysis of various components in Cremophor® EL and development of a compound with reduced toxicity, Lorenz W. et al. (1982) Agents and Actions, vol. 12, 1/2) discuss anaphylactic reactions in man following administration of drugs solubilized with Cremophor® EL (polyethylenglycolglycerol riconoleate).

EP2279728A1, priority 31.07.2009, published on 02.02.2011 after the priority date of this application, describes solid dosage forms of HIV protease inhibitors. Example 5 discloses a dosage form comprising lopinavir, ritonavir, stearic acid and EUDRAGIT® E PO. which differs from the dosage form as claimed herein.

### Objects of the invention

The need to provide pharmaceutical dosage form for antiretroviral active ingredients with high bioavailabilty often requires the formation of solid dispersions or solid solutions for instance by thermal processing. This has the disadvantage that the stability of the antiretroviral active ingredients may be affected by chemical degradation. On the other hand additional excipients like pharmaceutically acceptable surfactants with an HLB value from 12 to 18 may be required to ensure high bioavailability without or with reduced chemical degradation.

One of the objects of the present invention is provide a pharmaceutical dosage form for antiretroviral active ingredients with improved solubility, high bioavailability without significant chemical degradation during thermal processing without the addition of undesired excipients.

The object is solved by a pharmaceutical dosage form comprising one or more antiretroviral active ingredients in the form of a solid dispersion or solid solution in a matrix, wherein said matrix comprises an amino(meth)acrylate copolymer, characterized in that the matrix does not contain any essential amounts of pharmaceutically acceptable surfactants with an HLB value from 12 to 18 and in that the matrix comprises a mono carboxylic acid or an alcohol with 12 to 22 carbon atoms or both, wherein the
pharmaceutical dosage form is containing
   5 to 25 % by weight lopinavir.
   1 to 10 % by weight ritonavir.
   5 to 20 % by weight stearic acid.
   50 - 80 % by weight amino methacrylate copolymer (USP/NF),
   up to 20 % by weight further pharmaceutically acceptable excipients,
whereby the components add up to 100 %.

### Detailed description of the invention

The invention refers to a pharmaceutical dosage form comprising one or more antiretroviral active ingredient in the form of a solid dispersion or solid solution in a matrix, wherein said matrix comprises an amino(meth)acrylate copolymer, characterized in that the matrix does not contain pharmaceutically acceptable surfactants with an HLB value from 12 to 18 and in that the matrix comprises a mono carboxylic acid or an alcohol with 12 to 22 carbon atoms or both within the further limitations of Claim 1.

### Antiretroviral active ingredients

Antiretroviral active ingredients mean such active pharmaceutical ingredients which may be used in the therapy of diseases caused by retroviruses, such as AIDS or HIV respectively. The term antiretroviral active ingredients shall include corresponding pharmaceutically acceptable salts, solvates or hydrates or any combinations of antiretroviral active ingredients.

Antiretroviral active ingredients may be divided in classes such as nucleoside-analoga, nucleoside-analogous reverse transcriptase-inhibitors (NRTI), non-nucleoside reverse transcriptase-inhibitors (NNRTI), proteinase-inhibitors (PI), entry-inhibitors, integrase-inhibitors, boosters and combination-preparations of antiretroviral active ingredients for high active anti-retroviral therapy (HAART).

Preferably the antiretroviral active ingredient belongs to the BCS class III or to the BCS class IV (Biopharmaceutical classification system according to Prof. Amidon; Amidon et al., Pharm. Res. 12, 413 - 420 (1995)).

The antiretroviral active ingredients may have a molecular weight M_{w} (M_{w}= molecular weight average) of 250 to 1000, preferably 500 to 800 g/mol. The M_{w} of ritonavir is for example 721 g/mol, the M_{w} of lopinavir is for example 629 g/mol.

Preferably the active antiretroviral substance is an antiretroviral proteinase inhibitor. For example ritonavir is used to inhibit a particular liver enzyme that normally metabolizes protease inhibitors, cytochrome P450-3A4 (CYP3A4). The drug's molecular structure inhibits CYP3A4, so a low dose can be used to enhance other protease inhibitors. The proteinase inhibitor ritonavir may be also be describes as a booster substance since its boosts the activity of other antiretroviral proteinase inhibitors when it is present in combination preparations such as Kaletra®. The active ingredient is lopinavir in combination with ritonavir or their corresponding pharmaceutically acceptable salts, solvates or hydrates or mixtures of these active ingredients.

### BCS Classes III and IV

Preferably the antiretroviral active ingredient may belong to the group of BCS classes III and IV (Biopharmaceutical classification system according to Prof. Amidon; (Amidon et al., Pharm. Res. 12, 413 - 420 (1995)).
BCS Class III - Low Permeability, High Solubility
The absorption is limited by the permeation rate but the drug is solvated very fast.
BCS Class IV - Low Permeability, Low Solubility
Those compounds have a poor bioavailability. Usually they are not well absorbed over the intestinal mucosa and a high variability is expected.

The active ingredient(s) of BCS classes III and IV has/have preferably a permeability which is less than 90 % of the administered dose based on a mass-balance determination or in comparison to and intravenous dose. Permeability is based indirectly on the extent of absorption of a drug substance in humans and directly on the measurement of rates of mass transfer across human intestinal membrane. Alternatively non-human systems capable of prediction the drug absorption systems capable of predicting the drug absorption in humans can be used (such as in-vitro culture methods). A drug substance is considered highly permeable when the extent of absorption in humans is determined to be 90 % or more of the administered dose based on a mass-balance determination or in comparison to and intravenous dose.

The active ingredients of BCS class IV may have solubilities in demineralized water of 3.3 g/l (20°C) or less. The active ingredients of BCS class III have good solubility in water. The active ingredients of BCS class IV have a low permeability. The advantages of the invention are therefore displayed in particular for the active ingredients of BCS class IV, since solubility and permeability of the active ingredient constitute the limitation of its bioavailability.

### Solubility in water

The active ingredients may have a solubility in demineralized water of 3.3 g/l or less, preferably 3.3 g/l or less, in particular 1.1 g/l or less.

The solubility in water for the active ingredient can be defined according to DAB 10 (Deutsches Arzneibuch [German Pharmacopoeia], 10th edition with 3rd revision 1994, Deutscher Apothekerverlag, Stuttgart and Govi Verlag, Frankfurt am Main, 2nd revision (1993), IV Allgemeine Vorschriften [IV General methods], p. 5 - 6, "Löslichkeit und Lösungsmittel" ["Solubility and solvents"]; see also Ph. Eur. 4.07, 2004).

### Examples for antiretroviral active ingredients

### Therapeutical classes:

1. Non nucleotide reverse transcriptase inhibitors (NNRTI):
   Delavirdine, Efavirenz, Etravirine, Ibacitabine, Loviride, Nevirapine
2. Nucleotide reverse transcriptase inhibitors (NRTI):
   Abacavir, Adefovir, Apricitabine, Cidofovir, Cytarabine, Didanosine, Emtricitabine, Entecavir, Famiciclovir, Fomovirsen, Idoxuridine, Lamivudine, Moroxydine, Penciclovir, Ribavirin, Stavudine, Telbivudine, Tenofovir, Tenofovir disoproxil, Trifluridine, Vidarabine, Viramidine, Zalcitabine, Zidovudine
3. Protease inhibitors (PI):
   Amprenavir, Atazanavir, Boceprevir, Darunavir, Fosamprenavir, Indinavir, Lopinavir, Nelfinavir Palinavir, Ritonavir, Saquinavir, Tipranavir
4. Integrase Inhibitors (INI)
   Ampligen, Elvitegravir, Raltegravir
5. Entry- or Fusioninhibitors (FI):
   Arbidol, Docosanol, Enfuvirtide, Maraviroc, Pleconaril, Raltegravir, Rimatidine, Tromantadine, Vicriviroc.

APIs, single or combinations, include their pharmaceutically acceptable salts, solvates, hydrates, enantiomers, derivatives, polymorphs or prodrugs

The antiretroviral active ingredient as claimed is lopinavir in combination with ritonavir or their corresponding pharmaceutically acceptable salts, solvates or hydrates or combinations thereof.

Pharmaceutical dosage forms, wherein lopinavir and ritonavir are combined, comprised or contained as active ingredients are claimed in Claim 1. The weight ratio of lopinavir to ritonavir is preferably from the ratio 8 to1 to the ratio 1:1, most preferred from the ratio 4 to 1 to the ratio 2 to 1 or even more preferred from the ratio 3.5 to 1 to the ratio 2.5 to 1.

### Solid dispersions and solid solutions

The term solid dispersion defines a solid state system comprising at lease two components wherein one component is dispersed throughout the other component or components. In the sense of the present invention one or more antiretroviral active ingredients are dispersed in a matrix, wherein said matrix comprises an amino(meth)acrylate copolymer.

The dispersion may be a purely physical uniformly distribution of the one or more antiretroviral active ingredients in the matrix, which means the active ingredient is present in the crystallized form as it was before it was mixed into the matrix structure. The still crystallized form of the active ingredient may be proven by X-ray differential scanning analysis. Particles of the active ingredient in crystal structures may be proven by scanning electron microscopy.

The dispersion may be a physical uniformly distribution of the one or more antiretroviral active ingredients in the matrix, wherein the active ingredient is the state of a amorphous particulate state of a solid dispersion. This means that the active ingredient has been transited from a crystallized state to a higher energy non-crystallized, amorphous state. The non-crystallized form of the active ingredient may be proven by X-ray differential scanning analysis which shows an altered spectrum. Particles of the active ingredient in non-crystal structures may be proven by scanning electron microscopy. The mean size of such particles, measured in length or in diameter, may be typically less than 500 µm in size, for instance not more than 100, not more than 10 or not more than 1 µm.

The dispersion may be a physical and chemically uniformly distribution of the one or more antiretroviral active ingredients in the matrix on the molecular level, which is called a solid solution. This means that the active ingredient has been transited from a crystallized state to a higher energy non-crystallized, molecular state. The non-crystallized form of the active ingredient may be proven by X-ray differential scanning analysis which shows an altered spectrum. The higher energy state of the solid solution may be proven by scanning electron microscopy showing the absence of particulate active ingredient structures.

Pharmaceutical dosage forms wherein the active ingredient is the state of a solid dispersion in an amorphous particulate state is most preferred.
In contrast to the solid dispersion state where the active ingredient is still present in comparably low energy but highly stable crystal form the solubility and thus the bioavailability of the active ingredient is increased in a solid dispersion in an amorphous particulate state.
In contrast to the solid solution state where the active ingredient is present in molecular distributed form the stability of the energy state is apparently better since the energy state is supposed to be in a lower level than in the solid solution state which tends to fall back to the crystallized state.

### Amino(meth)acrylate copolymer

Pharmaceutical dosage form according to the invention comprises an amino(meth)acrylate copolymer.

The amino(meth)acrylate copolymer may be composed partly or fully of alkyl acrylates and/or alkyl methacrylates having a tertiary amino group in the alkyl radical. Suitable (meth)acrylate copolymers are known, for example, from EP 0 058 765 B1.

Suitable monomers with functional tertiary amino groups are detailed in US 4 705 695, column 3 line 64 to column 4 line 13. Mention should be made in particular of dimethylaminoethyl acrylate, 2-dimethylaminopropyl acrylate, dimethylaminopropyl methacrylate, dimethylaminobenzyl acrylate, dimethylaminobenzyl methacrylate, (3-dimethylamino-2,2-dimethyl)propyl acrylate, dimethylamino-2,2-dimethyl)propyl methacrylate, (3-diethylamino-2,2-dimethyl)propyl acrylate and diethylamino-2,2-dimethyl)propyl methacrylate. Particular preference is given to dimethylaminoethyl methacrylate.

The amino(meth)acrylate copolymer may be a copolymer composed of 30 to 80% by weight of C₁- to C₄-alkyl esters of acrylic or of methacrylic acid, and 70 to 20% by weight of alkyl(meth)acrylate monomers having a tertiary amino group in the alkyl radical.

The amino(meth)acrylate copolymer may be a copolymer composed of 20 - 30% by weight of methyl methacrylate, 20 - 30% by weight of butyl methacrylate and 60 - 40% by weight of dimethylaminoethyl methacrylate.

A specifically suitable commercial amino (meth)acrylate copolymer is, for example, formed from 25% by weight of methyl methacrylate, 25% by weight of butyl methacrylate and 50% by weight of dimethylaminoethyl methacrylate (EUDRAGIT® E100 or EUDRAGIT® E PO (powder form)). EUDRAGIT® E100 and EUDRAGIT® E PO are water-soluble below approx. pH 5.0 and are thus also gastric juice-soluble.

Suitable copolymers may be the "amino methacrylate copolymer (USP/NF)", "basic butylated methacrylate copolymer (Ph. Eur)" or "aminoalkyl Methacrylate Copolymer E (JPE)" which are of the EUDRAGIT® E type.

### Pharmaceutically acceptable surfactants with an HLB value from 12 to 18

WO2008/067164A2 describes solid pharmaceutical dosage forms comprising a solid dispersion or solid solution of ritonavir in a matrix. The matrix comprises at least one pharmaceutical acceptable water-soluble polymer and at least one pharmaceutically acceptable surfactant. The pharmaceutically acceptable surfactant has an HLB value of from 12 to 18. However it has been found that at least some pharmaceutically acceptable surfactants with an HLB value from 12 to 18 may show undesired biological effects such as anaphylactic hypersensitivity reactions, hyperlipidermia, abnormal lipoprotein pattern, aggregation of erythrocytes and peripheral neuropathy (Cremophor® EL: the drawbacks and advantages of vehicle selection for drug fomulation (2001) Gelderblom, H., Verweij J. and Sparreboom A., European Journal of Cancer, 37, 1590 - 1598*;*
Histamine release and hyposensitive reactions in dogs by solubilizing agents and fatty acids: Analysis of various components in Cremophor® El and development of a compound with reduced toxicity, Lorenz W. et al. (1982) Agents and Actions, vol. 12, 1/2).

The invention defines that the matrix does not contain any essential amounts of pharmaceutically acceptable surfactants with an HLB value from 12 to 18. Any essential amounts of pharmaceutically acceptable surfactants with an HLB value from 12 to 18 may be such amounts that do not effect the chemical degradation of the one or more antiretroviral active ingredients during the processing to the solid dispersion or solid solution state to be more than 15% or more than 10% in relation to the total amount of the one or more antiretroviral active ingredients that were initially incorporated (A suitable analytical method to detect degradation products is liquid chromatography, see for instance Indian Pharmacopeia, Assay Methods described in the monographs). Any essential amounts of pharmaceutically acceptable surfactants with an HLB value from 12 to 18 may be amounts of less than 5, less than 4, less than 3, less than 2 or less than 1 % by weight in relation to the total weight of the matrix.

Thus the pharmaceutical dosage form according to the invention contains 15 % by weight or less, preferably 10 % by weight or less chemical degradation products of initially incorporated one or more antiretroviral active ingredients.

### Mono carboxylic acids or alcohols with 12 to 22 carbon atoms.

The pharmaceutical dosage form according to the invention may comprise a mono carboxylic acid with 12 to 22 carbon atoms or an alcohol with 12 to 22 carbon atoms within the further limitations of Claim 1. Mono carboxylic acids or an alcohols with 12 to 22 carbon atoms are as a rule poorly soluble in water at 25 °C which means that a solubility usually of less than 1mg/ml, of less than 0.5 mg/ml, of less than 0.1mg/ml. of less than 0.01 mg/ml in water.

In the particular embodiment of the present invention the mono carboxylic acid with 12 to 22 carbon atoms is stearic acid.

### Further pharmaceutically acceptable excipients

Pharmaceutical dosage form according to the invention may comprise further pharmaceutically acceptable excipients which may be selected from the classes of antioxidants, brighteners, flavouring agents, flow aids for instance silicates like fumed or precipitated silica, fragrances, glidants (release agents), penetration-promoting agents, pigments, polymers which are not amino(meth)acrylate copolymers, pore-forming agents or stabilizers.

Further pharmaceutically acceptable excipients are well known to the skilled person. Such excipients may be contained for practical reasons, for instance to avoid stickiness or to add a colour. However these excipients usually do not contribute or do show any or almost no effect on the invention itself as claimed here. They may be used as processing adjuvants and are intended to ensure a reliable and reproducible preparation process as well as good long-term storage stability, or they achieve additional advantageous properties in the pharmaceutical form.

### Pharmaceutical dosage form

The pharmaceutical dosage form according to the invention is containing
5 to 25, 10 to 20 % by weight lopinavir,
1 to 10, 2 to 8 % by weight ritonavir,
5 to 20, 8 to 15 % by weight stearic acid
50 - 80, 55 - 70 % by weight amino methacrylate copolymer (USP/NF),
up to 20, up to 10, 1 to 20 % by weight further pharmaceutically acceptable excipients, preferably 2 to 10 % by weight microcrystalline cellulose and 0,5 to 4 % by weight fumed silica (Aerosil®)
whereby all components add up to 100 %.

### Process for producing a pharmaceutical dosage form

A suitable process for producing a pharmaceutical dosage form according to the invention is the melt extrusion technique. Preferred temperature level are from 50 to 180 °C.

The melt extrusion process is preferred over the solvent process, one reason being that the handling of solvents, which is problematic for procedural, health protection and environmental protection reasons, is dispensed with.

The melt extrusion process may be performed with the aid of an extruder, especially by means of a twin-screw extruder. It is favourable when the extruder or the twin-screw extruder is equipped with a degassing zone. The water-soluble and the water-insoluble polymer can be incorporated as a solid, as a polymer solution or as a polymer dispersion. The active ingredient can be added as a solid, as a solution or as a suspension. The extrudate is preferably processed by means of strand granulation and hot-cut methods to give cylindrical, elongated strand granules, or by hot-cutting with cooling to give rounded pellets. EP 1 563 987 A1 describes a suitable apparatus for producing rounded pellets (pelletizer). Granules can preferably be ground to powders with, for example, particle sizes of less than/equal to 1 mm, preferably in the range of 50 to 500 µm.

A pharmaceutical dosage form prepared by the melt extrusion process may be further processed to granules, pellets or powders, if appropriate formulated by means of pharmaceutically customary excipients, and processed in a manner known per se, for example by mixing, compressing, powder layering and/or encapsulation to a pharmaceutical form, for example to tablets, or preferably to a multiparticulate pharmaceutical form, especially to pellet-containing tablets, minitablets, capsules, sachets or reconstitutable powders. Pellets or tablets may be coated by film forming polymers by the spray coating technique in order to achieve sustained release or gastric resistant or enteric properties.

### Examples

### Materials and Methods

The following ingredients were used for the trials involved in the study.

| **Ingredient** | **Manufacturer** | **Batch No.** |
|---|---|---|
| Lopinavir | Hetero drugs | LO0090808 |
| Ritonavir | Hetero drugs | RI0090608 |
| EUDRAGIT® EPO | Evonik Industries | G070831112 |
| Kollidon® VA 64 | BASF | 76016347GO |
| Collodial Silicon dioxide (Aerosil® 200) | Evonik Industries | 3157050514 |
| Sodium lauryl sulphate | Stepan Co. | 7295746 |
| Stearic Acid | Stearinerie Dubois | 07041073 |
| Citric Acid | Merck | MB8M580394 |
| Tartaric acid | Merck | MF8M571497 |

The following equipment is used for the trials

| **Name of the equipment** | **Model** |
|---|---|
| Melt Extruder | Thermo ScientificPharma HME 16 |
| HPLC | Waters Alliance 2695 and Agilent 1100 series |

### Formulation methodology

The melt extrusion was carried out on Thermo Scientific Pharma HME 16, double screw co-rotating assembly with 10 heating zones and a 1.0 mm pellet cutter. Lopinavir, Ritonavir and the polymers were weighed and sifted through a sieve of 20 mesh (0.84 mm) and blended in a polybag manually. This was loaded on to the hopper and the extrusion was carried out at a feed rate of 0.2 to 0.8 kg/hour and screw speed of 75 to 150 rpm. Maximum temperatures were adjusted to 105, 125 and 150° C

### Analytical methodology

### Drug release:

| | |
|---|---|
| Apparatus: | USP Type- II (Paddle) |
| Medium: | 0.06 M Polyoxyethylene 10-lauryl ether in 0.1N HCI / Acetate buffer pH 4.5 with 0.06 M Polyoxyethylene 10-lauryl ether |
| Dissolution volume: | 900 ml. |
| Temperature: | 37 ± 0.5 ° C. |
| Rotation speed: | 75 rpm |
| Withdrawal volume: | 10 ml with replenishment |
| Withdrawal interval: | 5, 10, 15, 30, 45, 60, 75, 90, 105 and 120 minutes |

### Detection:

| | |
|---|---|
| Mode: | HPLC, λmax : 210nm |
| Column : | Phenomenex C8, 150 mm X 4.6 mm, 5 µ particle size |
| Mobile phase : | Buffer: Acetonitrile: Methanol (45: 44: 11 v/v) |
| Buffer: | Phosphate buffer, pH 3. |
| Column temperature: | 250C |
| Injection volume : | 10 µl |

### Example 1 and 2: Drug Polymer Ratios : 1:1 and 1:2

Drug / polymer ratios: 1:1 and 1:2, batch size 300 g

**Table 1: Formulations**

| **Ingredients** | **content (% by weight)** | |
|---|---|---|
| | **Example 1 Batch No. 17 Drug:Polymer (1:1)** | **Example 2 Batch No. 05 Drug:Polymer (1:2)** |
| Lopinavir | 34.48 | 21.98 |
| Ritonavir | 8.62 | 5.49 |
| EUDRAGIT® EPO | 43.10 | 54.95 |
| Stearic acid | 6.47 | 8.24 |
| Sodium Lauryl Sulphate | 3.02 | 3.85 |
| Aerosil® 200 | 4.31 | 5.49 |
| Total | 100 | 100 |

### Comparative examples 3 and 4

Drug / polymer ratios: 1:1 and 1:2, batch size 300 g

**Table 2: Formulations**

| **Ingredients** | **% content** | |
|---|---|---|
| | **Example 3 Batch No. 18 Drug:Polymer (1:1)** | **Example 4 Batch No. 06 Drug: Polymer (1:2)** |
| Lopinavir | 34.48 | 21.98 |
| Ritonavir | 8.62 | 5.49 |
| Kollidon® VA 64 | 43.10 | 54.95 |
| Stearic acid | 6.47 | 8.24 |
| Sodium Lauryl Sulphate | 3.02 | 3.85 |
| Aerosil® 200 | 4.31 | 5.49 |
| Total | 100 | 100 |

The mixtures of examples 1 to 4 were extrudated, comminuted and subsequently sifted through a sieve of 40 mesh (0.42 mm) and analyzed. X-ray diffraction analysis was carried out for crytallinity. Dissolution rate analysis of both active compound was carried out to check solubility enhancement.

### Crystallinity anaysis by X-ray diffraction (XRD)

- The XRD pattern of pure Lopinavir and pure Ritonavir API shows that the drug originally exists in crystalline form.
- The extrudates of Lopinavir and Ritonavir of examples 2 and 4 after melt extrusion show formation of the amorphous form.

### In Vitro dissolution rate of the extruded mixtures of examples 1 and 3 (Batch no. 17 & 18, Drug: Polymer in ratios 1:1)

**Table 3: Ritonavir solubility in 0.1 N HCI**

| **Time in mins.** | **Example 1 Batch no. 17** | **Example 3 Batch no. 18** |
|---|---|---|
| | **Release in %** | **Release in %** |
| 0 | 0 | 0 |
| 5.0 | 96.4 | 31.2 |
| 10.0 | 98.5 | 38.8 |
| 15.0 | 98.4 | 44.2 |
| 30.0 | 98.7 | 56.1 |
| 45.0 | 98.6 | 64.2 |
| 60.0 | 98.7 | 70.2 |
| 90.0 | 99.0 | 78.7 |
| 120.0 | 99.7 | 83.9 |

**Table 4: Lopinavir release rate in 0.1N HCl**

| **Time in mins.** | **Example 1 Batch no. 17** | **Example 3 Batch no. 18** |
|---|---|---|
| | **Release in %** | **Release in %** |
| 0 | 0 | 0 |
| 5.0 | 90.9 | 24.8 |
| 10.0 | 93.5 | 32.0 |
| 15.0 | 93.4 | 37.2 |
| 30.0 | 93.6 | 48.8 |
| 45.0 | 93.4 | 57.1 |
| 60.0 | 93.4 | 63.5 |
| 90.0 | 93.5 | 72.5 |
| 120.0 | 94.1 | 78.2 |

**Table 5: Ritonavir release rate in acetate buffer pH 4.5**

| **Time in mins.** | **Example 1 Batch no. 17** | **Example 3 Batch no. 18)** |
|---|---|---|
| | **Release in %** | **Release in %** |
| 0 | 0 | 0 |
| 5.0 | 61.1 | 30.2 |
| 10.0 | 71.2 | 42.0 |
| 15.0 | 78.0 | 45.1 |
| 30.0 | 89.7 | 58.2 |
| 45.0 | 95.0 | 68.1 |
| 60.0 | 97.8 | 75.2 |
| 90.0 | 99.6 | 85.1 |
| 120.0 | 100.2 | 91.6 |

**Table 6: Lopinavir release rate in acetate buffer pH 4.5**

| **Time in mins.** | **Example 1 Batch no. 17** | **Example 3 Batch no. 18** |
|---|---|---|
| | **Release in %** | **Release in %** |
| 0 | 0 | 0 |
| 5.0 | 63.3 | 32.8 |
| 10.0 | 73.3 | 45.5 |
| 15.0 | 80.0 | 48.5 |
| 30.0 | 91.5 | 62.7 |
| 45.0 | 97.0 | 73.0 |
| 60.0 | 99.5 | 80.5 |
| 90.0 | 101.5 | 91.0 |
| 120.0 | 102.3 | 97.4 |

### In Vitro dissolution rate of the extruded mixtures of the examples 2 and 4 (Batch no. 05 & 06; Drug: Polymer in ratios 1:2)

**Table 7: Ritonavir release rate in 0.1N HCl**

| **Time in mins.** | **Example 2 Batch no. 05** | **Example 4 Batch no. 06** |
|---|---|---|
| | **Release in %** | **Release in %** |
| 0 | 0 | 0 |
| 5.0 | 96.7 | 81.6 |
| 10.0 | 99.7 | 95.3 |
| 15.0 | 100.3 | 97.2 |
| 30.0 | 100.4 | 97.8 |
| 45.0 | 100.6 | 98.3 |
| 60.0 | 101.4 | 98.5 |
| 90.0 | 101.6 | 99.3 |
| 120.0 | 102.9 | 100.4 |

**Table 8: Lopinavir release rate in 0.1N HCl**

| **Time in mins.** | **Example 2 Batch no. 05** | **Example 4 Batch no. 06** |
|---|---|---|
| | **Release in %** | **Release in %** |
| 0 | 0 | 0 |
| 5.0 | 90.6 | 67.2 |
| 10.0 | 96.9 | 92.1 |
| 15.0 | 98.9 | 96.6 |
| 30.0 | 99.2 | 98.2 |
| 45.0 | 99.6 | 98.9 |
| 60.0 | 100.5 | 99.7 |
| 90.0 | 100.6 | 99.0 |
| 120.0 | 100.9 | 100.0 |

**Table 9: Ritonavir release rate in acetate buffer pH 4.5**

| **Time in mins.** | **Example 2 Batch no. 05** | **Example 4 Batch no. 06)** |
|---|---|---|
| | **Release in %** | **Release in %** |
| 0 | 0 | 0 |
| 5.0 | 94.5 | 93.2 |
| 10.0 | 101.1 | 97.8 |
| 15.0 | 101.0 | 97.9 |
| 30.0 | 101.6 | 97.9 |
| 45.0 | 101.5 | 98.0 |
| 60.0 | 102.1 | 98.9 |
| 90.0 | 102.8 | 99.9 |
| 120.0 | 104.0 | 100.8 |

**Table 10: Lopinavir release rate in acetate buffer pH 4.5**

| **Time in mins.** | **Example 2 Batch no. 05** | **Example 4 Batch no. 06** |
|---|---|---|
| | **Release in %** | **Release in %** |
| 0 | 0 | 0 |
| 5.0 | 96.2 | 97.7 |
| 10.0 | 102.1 | 101.7 |
| 15.0 | 101.9 | 101.7 |
| 30.0 | 102.5 | 101.7 |
| 45.0 | 102.3 | 101.7 |
| 60.0 | 102.9 | 102.8 |
| 90.0 | 103.7 | 103.8 |
| 120.0 | 104.9 | 104.8 |

### Discussion of results:

At a 1:1 ratios of drug: polymer, extrudates with EUDRAGIT® EPO showed a drug release rate improvement in comparison to extrudates with Kollidon® VA 64 extrudates in both dissolution media 0.1 N HCI and acetate buffer pH 4.5. At a 1: 2 ratio of drug polymer content both the release rates were accelerated but almost equivalent.

### Examples 5A, 5B, 5C, 6, 7, and 8 (Chemical stability)

Polymer: Drug ratios: 1:5, Batch size: 200g

**Table 11: Formulations**

| **Ingredients** | **content (% by weight)** | | | |
|---|---|---|---|---|
| | **Example 5A, 5B, 5C Batch No. 24, 28 and 29 Drug: EUDRAGIT® EPO with tartaric acid** | **Example 6 Batch No. 25 Drug: Kollidon® VA 64 with Stearic acid and SLS** | **Example 7 Batch No. 26 Drug: EUDRAGIT® E PO with citric acid acid and PEG 3350** | **Example 8 Batch No. 27 Drug: EUDRAGIT® E PO with stearic acid and SLS** |
| Ritonavir | 11.76 | 13.16 | 9.52 | 13.16 |
| EUDRAGIT® E PO | 58.82 | -- | 47.62 | 65.79 |
| Kollidon® VA 64 | -- | 65.79 | -- | -- |
| Stearic acid | 11.76 | 9.87 | -- | 9.87 |
| Tartaric acid | 11.76 | -- | -- | -- |
| Citric acid | -- | -- | 23.81 | -- |
| Sodium Lauryl Sulphate | -- | 4.61 | -- | 4.61 |
| PEG 3350 | -- | -- | 14.29 | -- |
| Aerosil® 200 | 5.89 | 6.58 | 4.76 | 6.58 |
| Total | 100 | 100 | 100 | 100 |

The mixtures of the examples 5A, 6, 7 and 8 were extruded at a maximum temperature of 150 °C. The mixtures of examples 5B and 5C are identical to example 5A but were extruded at a maximum temperature of 125 °C or 105 °C respectively.

The extruded mixtures were comminuted and subsequently sifted through a sieve of 40 mesh (0.42 mm) and analyzed for degradation products of ritonavir.

### Analytical Methodology to detect degradation products:

### Chromatographic conditions (Ref: Indian Pharmacopoeia. Assay method)

Detection : 210nm
Column : Agilent C8, 150 mm X 4.6 mm, 5 µ particle size
Mobile phase : Buffer: Acetonitrile: Methanol (45: 44: 11 v/v)
Buffer : Phosphate buffer, pH 3, 6.8g of Potassium dihydrogen phosphate dissolved in 1Litre water and pH adjusted to 3.0 with OPA.
Flow : 1 ml/min;
Column temperature : 25 °C;
Injection volume : 10 µl

### Sample Preparation:

Sample containing around 50 mg of ritonavir was weighed accurately and transferred into a 50 ml volumetric flask. About 40 ml of the mobile phase was added into the flask and sonicated for 30 minutes. Volume was made up to 50 ml with mobile phase. This 1000 ppm ritonavir solution was used as the sample solution.
Estimation of impurities was done using 10 ppm of ritonavir standard solution.

**Table 12: Results of the chemical degradation assays**

| **Example / Batch No.** | **Total Impurities** |
|---|---|
| 8 / 27 | 8.0 % |
| 7 / 26 | 41.0 % |
| 5A / 24 | 49.8 % |
| 6 / 25 | 17.4 % |
| 5B / 28 | 17.2 % |
| 5C / 29 | 16.7 % |

### Conclusions

Degradation of examples 5A and 7 (EUDRAGIT® E with stearic acid/tartaric acid or citric acid at 150 °C) was more than 40 % indicating that the addition of water soluble acids cause instability of ritonavir at high temperature.

Degradation of examples 5B, 5C and 6 (EUDRAGIT® E with stearic acid/tartaric acid, at 125 °C and 105 °C and Kollidon® VA64/stearic acid at 150 °C) was more around 17 % indicating that the addition of water soluble acids cause instability even at lower temperatures and that the addition of water insoluble acid causes instability in the presence of a water soluble polymer (Kollidon® VA64).

Degradation of example 8 (EUDRAGIT® E/stearic acid) was less than 10 % indicating that the addition of water insoluble acids cause to a water insoluble polymer maintains stability of ritonavir even at high temperature.

### Examples 9 and 10 (intermediate samples for in-vivo bioavailability testing)

**Table 13: Formulation**

| **Ingredients** | **Drug: EUDRAGIT® E PO (1:3) Example 9 / Batch no. 69** | |
|---|---|---|
| | **% Content** | **Batch Quantity (g)** |
| Lopinavir | 16.53 | 165.29 |
| Ritonavir | 4.13 | 41.32 |
| EUDRAGIT® EPO | 61.98 | 619.83 |
| Stearic acid (micronised) | 9.30 | 61.98 |
| Microcrystalline cellulose (Avicel® PH 101) | 6.20 | 92.98 |
| Aerosil® 200 | 1.86 | 18.60 |
| **Total** | 100.00 | 1000.0 |

**Table 14: Formulations**

| **Ingredients** | **Drug: EUDRAGIT® E PO (1:3) Example 10 / Batch no. 68** | |
|---|---|---|
| | **% Content** | **Batch Quantity (g)** |
| Lopinavir | 18.2 | 182.2 |
| Ritonavir | 4.6 | 45.6 |
| Kollidon® VA64 | 68.3 | 683.4 |
| Microcrystalline cellulose (Avicel® PH 101) | 6.8 | 68.3 |
| Aerosil® 200 | 2.1 | 20.5 |
| **Total** | 100.0 | 1000.0 |

The mixtures of the examples 9 and 10 were extruded at a maximum temperature of 125 °C. The extruded mixtures were comminuted and subsequently sifted through a sieve of 40 mesh (0.42 mm). The sifted powders were further processed to mixtures which could be filled in capsules.

### Examples 11 and 12 (samples for in-vivo bioavailability testing)

The extrudates of examples 9 and 10 were milled (comminuted) and the fraction of 180- 425 microns was collected and mixed with further excipients to give the preparations of examples 11 and 12 respectively (s. table 15).

**Table 15: Formulations**

| **Ingredients** | **Weight per capsule (mg)** | |
|---|---|---|
| | **Example 11/ batch no. 69** | **Example 12/ batch no. 68** |
| Extrudate of example 9 | 302.5 | - |
| Extrudate of example 10 | - | 274.4 |
| Microcrystalline cellulose (Avicel® PH 101) | - | 28.1 |
| Crospovidone (Kollidon® CL) | 16.0 | 16.0 |
| Aerosil® 200 | 3.0 | 3.0 |
| Net content | 321.5 | 321.5 |

The ingredients of table 15 were mixed and subsequently filled in gelatine capsules of size 0.

The capsules were tested for their dissolution rates in water as described in Office of Generic Drugs (OGD) of the US Food and Drug Administration (FDA).

**Table 16: Dissolution rate of ritonavir in water**

| **Time [min]** | **Capsule example 11 Release in %** | **Capsule example 12 Release in %** |
|---|---|---|
| 0 | 0 | 0 |
| 5 | 8 | 4 |
| 10 | 11 | 12 |
| 15 | 16 | 18 |
| 30 | 43 | 32 |
| 45 | 52 | 43 |
| 60 | 60 | 51 |
| 90 | 69 | 62 |
| 120 | 81 | 71 |

**Table 17: Dissolution rate of lopinavir in water**

| **Time [min]** | **Capsules example 11 Release in %** | **Capsules example 12 Release in %** |
|---|---|---|
| 0 | 0 | 0 |
| 5 | 9 | 6 |
| 10 | 21 | 13 |
| 15 | 32 | 18 |
| 30 | 48 | 35 |
| 45 | 57 | 46 |
| 60 | 63 | 54 |
| 90 | 71 | 66 |
| 120 | 86 | 78 |

Results: The dissolution rate of the capsules from example 11 (EUDRAGIT® EPO) and the capsules from example 12 (Kollidon® VA64) were approximately comparable and confirmed the general suitability for in-vivo studies.

### In vivo bioavailability testing

13 healthy human volunteers were administered with 4 capsules of the capsules from example 11 (EUDRAGIT® EPO) and the capsules from example 12 (Kollidon® VA64) as a single dose corresponding to a dose of 200mg Lopinavir/50mg Ritonavir. Blood samples were taken over a period of 48 hours and analysed for their Lopinavir/Ritonavir content.

The pharmacokinetic parameters from the blood levels analysed after ingestion of the capsules from example 12 (Kollidon® VA64) were beyond the detection levels.

The pharmacokinetic parameters from the blood levels analysed after ingestion of the capsules from example 11 (EUDRAGIT® EPO) are shown in tables 18 and 19.

**Table 18: Descriptive statistics for Pharmacokinetic parameters of Ritonavir**

| **Measures** | **Cₘₐₓ (ng/mL)** | **AUC₀₋ₜ (ng*hr/mL)** | **AUC_{0-inf} (ng*hr/mL)** | **Tₘₐₓ (hr)** |
|---|---|---|---|---|
| Mean | 89.16 | 590.5 | 1074.75 | 4.46 |

**Table 19: Descriptive statistics for Pharmacokinetic parameters of Lopinavir**

| **Measures** | **Cₘₐₓ (ng/mL)** | **AUC₀₋ₜ (ng*hr/mL)** | **AUC_{0-inf} (ng*hr/mL)** | **Tₘₐₓ (hr)** |
|---|---|---|---|---|
| Mean | 1943.95 | 16553.97 | 22414.46 | 4.04 |

Discussion: The pharmaceutical dosage form according to the invention provided in-vivo sufficient blood level concentrations of ritonavir and lopinavir. In contrast the non-inventive formulation using the water soluble polymer Kollidon® VA64 did not provide detectable blood level concentrations. Thus it appears that water soluble polymers as described in WO2008/067164A2 need to be formulated together with pharmaceutical acceptable surfactants with HLB-values of from 12 to 18 which can be omitted by using the present invention.

### Storage stability testing

The capsules from example 11 (EUDRAGIT® EPO) and the capsules from example 12 (Kollidon® VA64) were tested storage stability at elevated condition of 40 °C and 75% relative humidity over a peroid of 6 months.

### Description of results:

The assay of lopinavir and ritonavir extrudes at accelerated conditions (40°C/75%RH) at the end of three month and six months were within 90% to 105% for both the batches. Neither significant chemical degradation nor physical chances (for example recrystallization) were observed.

## Claims

1. A pharmaceutical dosage form comprising one or more antiretroviral active ingredients in the form of a solid dispersion or solid solution in a matrix, wherein said matrix comprises an amino(meth)acrylate copolymer, **characterized in that** the matrix does not contain any essential amounts of pharmaceutically acceptable surfactants with an HLB value from 12 to 18 and **in that** the matrix comprises a mono carboxylic acid or an alcohol with 12 to 22 carbon atoms or both, **wherein the**
**pharmaceutical dosage form is containing**
**5 to 25 % by weight lopinavir,**
**1 to 10 % by weight ritonavir,**
**5 to 20 % by weight stearic acid,**
**50** - **80 % by weight amino methacrylate copolymer (USP/NF),**
**up to 20 % by weight further pharmaceutically acceptable excipients,**
**whereby the components add up to 100 %.**

2. Pharmaceutical dosage form according to claim 1, wherein the active ingredient is the state of an amorphous particulate state of a solid dispersion.

3. Pharmaceutical dosage form according to claim 1 or 2, wherein the amino(meth)acrylate copolymer is a copolymer composed of 30 to 80% by weight of C₁- to C₄-alkyl esters of acrylic or of methacrylic acid, and 70 to 20% by weight of alkyl(meth)acrylate monomers having a tertiary amino group in the alkyl radical.

4. Pharmaceutical dosage form according to one or more claims 1 to 3, wherein the amino(meth)acrylate copolymer is a copolymer composed of 20 - 30% by weight of methyl methacrylate, 20 - 30% by weight of butyl methacrylate and 60 - 40% by weight of dimethylaminoethyl methacrylate.

5. Pharmaceutical dosage form according to one or more claims 1 to 4, wherein further pharmaceutically acceptable excipients selected from the classes of antioxidants, brighteners, flavouring agents, flow aids for instance silicates like fumed or precipitated silica, fragrances, glidants (release agents), penetration-promoting agents, pigments, polymers which are not amino(meth)acrylate copolymers, pore-forming agents or stabilizers are contained.

6. Pharmaceutical dosage form according to one or more claims 1 to 5 wherein 15 % or less chemical degradation products of initially incorporated one or more antiretroviral active ingredient(s) are contained.

7. Process for producing a pharmaceutical dosage form according to one or more claims 1 to 6 by melt extrusion technique at a temperature from 50 to 180 °C.

## Patentansprüche

1. Pharmazeutische Darreichungsform mit einem oder mehreren antiretroviralen Wirkstoffen in Form einer Feststoffdispersion oder Feststofflösung in einer Matrix, wobei die Matrix ein Amino(meth)acrylat-Copolymer umfasst,
**dadurch gekennzeichnet, dass** die Matrix keine wesentlichen Mengen pharmazeutisch akzeptabler oberflächenaktiver Mittel mit einem HLB-Wert von 12 bis 18 umfasst und dass die Matrix eine Monocarboxylsäure oder einen Alkohol mit 12 bis 22 Kohlenstoffatomen oder beides umfasst, wobei die pharmazeutische Darreichungsform
5 bis 25 Gew.-% Lopinavir,
1 bis 10 Gew.-% Ritonavir,
5 bis 20 Gew.-% Stearinsäure,
50 bis 80 Gew.-% Aminomethacrylat-Copolymer (USP/NF),
bis zu 20 Gew.-% weitere pharmazeutisch akzeptable Hilfsstoffe
enthält,
wobei die Bestandteile zusammen 100 % ergeben.

2. Pharmazeutische Darreichungsform nach Anspruch 1, wobei es sich bei dem Wirkstoff um den Zustand eines Amorphe-Partikel-Zustands einer Feststoffdispersion handelt.

3. Pharmazeutische Darreichungsform nach Anspruch 1 oder 2, wobei es sich bei dem Amino(meth)acrylat-Copolymer um ein Copolymer handelt, das zu 30 bis 80 Gew.-% aus C₁- bis C₄-Alkylestern von Acryl- oder von Methacrylsäure, und zu 70 bis 20 Gew.-% aus Alkyl(meth)acrylat-Monomeren mit einer tertiären Aminogruppe im Alkylradikal besteht.

4. Pharmazeutische Darreichungsform nach einem oder mehreren der Ansprüche 1 bis 3, wobei es sich bei dem Amino(meth)acrylat-Copolymer um ein Copolymer handelt, das zu 20 bis 30 Gew.-% aus Methylmethacrylat, zu 20 bis 30 Gew.-% aus Butylmethacrylat, und zu 60 bis 40 Gew.-% aus Dimethylaminoethylmethacrylat besteht.

5. Pharmazeutische Darreichungsform nach einem oder mehreren der Ansprüche 1 bis 4, wobei weitere pharmazeutisch akzeptable Hilfsstoffe, ausgewählt aus den Klassen der Antioxidantien, Aufheller, Geschmacksstoffe, Fließhilfen, zum Beispiel Silikate wie geräucherte oder gefällte Kieselerde, Duftstoffe, Fließregulierungsmittel (Trennmittel), penetrationsfördernden Mittel, Pigmente, Polymere außer Amino(meth)acrylat-Copolymeren, porenbildenden Mittel oder Stabilisatoren, enthalten sind.

6. Pharmazeutische Darreichungsform nach einem oder mehreren der Ansprüche 1 bis 5, wobei 15 % oder weniger chemische Abbauprodukte von dem/n ursprünglich eingearbeiteten einen oder mehreren antiretroviralen Wirkstoff(en) enthalten sind.

7. Verfahren zur Herstellung einer pharmazeutischen Darreichungsform nach einem oder mehreren der Ansprüche 1 bis 6 durch Schmelzextrusionstechnik bei einer Temperatur von 50 bis 180 °C.

## Revendications

1. Forme galénique comprenant un ou plusieurs principes actifs antirétroviraux sous la forme d'une dispersion solide ou d'une solution solide dans une matrice, où ladite matrice comprend un copolymère d'amino(méth)acrylate, **caractérisée en ce que** la matrice ne contient aucune quantité essentielle de tensioactifs pharmaceutiquement acceptables de valeurs de HLB comprises entre 12 et 18 et **en ce que** la matrice comprend un acide monocarboxylique ou un alcool comportant 12 à 22 atomes de carbone, ou les deux, où la forme galénique comporte
5 à 25 % en masse de lopinavir,
1 à 10 % en masse de ritonavir,
5 à 20 % en masse d'acide stéarique,
50 à 80 % en masse de copolymère d'aminométhacrylate (USP/NF),
jusqu'à 20 % en masse d'excipients pharmaceutiquement acceptables supplémentaires,
la somme des proportions des composants atteignant 100 %.

2. Forme galénique selon la revendication 1, où le principe actif est dans un état particulaire amorphe de dispersion solide.

3. Forme galénique selon la revendication 1 ou 2, où le copolymère d'amino(méth)acrylate est un copolymère composé de 30 à 80 % en masse d'esters d'alkyle en C₁ à C₄ d'acide acrylique ou méthacrylique, et de 70 à 20 % en masse de monomères d'alkyl(méth)acrylate comportant un groupement amino tertiaire au niveau du radical alkyle.

4. Forme galénique selon une ou plusieurs des revendications 1 à 3, où le copolymère d'amino(méth)acrylate est un copolymère composé de 20 à 30 % en masse de méthacrylate de méthyle, de 20 à 30 % en masse de méthacrylate de butyle et de 60 à 40 % en masse de méthacrylate de diméthylaminoéthyle.

5. Forme galénique selon l'une quelconque des revendications 1 à 4, où des excipients pharmaceutiquement acceptables supplémentaires choisis parmi les classes des antioxydants, azurants, arômes, agents rhéologiques, par exemple silicates comme la silice pyrogénée ou précipitée, parfums, agents glissants (agents de libération), agents favorisant la pénétration, pigments, polymères qui ne sont pas des copolymères d'amino(méth)acrylate, agents de formation de pores ou stabilisants sont inclus.

6. Forme galénique selon une ou plusieurs des revendications 1 à 5, où 15 % ou moins de produits de dégradation chimique d'un ou de plusieurs principes actifs antirétroviraux initialement incorporés sont inclus.

7. Procédé de production d'une forme galénique selon une ou plusieurs des revendications 1 à 6 par extrusion à l'état fondu à une température comprise entre 50 et 180 °C.
